# EUROPÄISCHE PATENTANMELDUNG

(11) **EP 0 670 318 A2**
(43) Veröffentlichungstag der Anmeldung: **06.09.1995**
(21) Anmeldenummer: 95102310.0
(22) Anmeldetag: 18.02.1995
(51) Int. Cl.: C07D 401/06, C07D 405/06, C07D 413/06, C07D 217/14, C07D 263/58, C07D 265/36, C07D 235/26, A61K 31/415, A61K 31/42, A61K 31/47, A61K 31/535

(54) **Acetamide mit analgetischer und neuroprotektiver Wirkung**

(30) Priorität: 03.03.1994 DE 4407047
(71) Anmelder: MERCK PATENT GmbH, D-64293 Darmstadt (DE)
(72) Erfinder: Gottschlich, Rudolf, Dr., D-64354 Reinheim (DE); Ackermann, Karl-August, D-64372 Ober-Ramstadt (DE); Seyfried, Christoph, Dr., D-64342 Jugenheim (DE); Barber, Andrew, Dr., D-64331 Weiterstadt (DE); Bartoszyk, Gerd, D-64331 Weiterstadt (DE); Greiner, Hartmut, Dr., D-64331 Weiterstadt (DE)

(57) **Zusammenfassung**

Neue Acetamide der Formel l 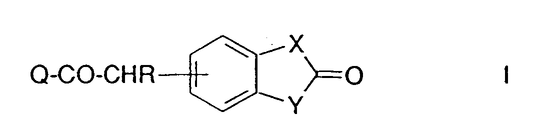
worin Q, R, X und Y die in Anspruch 1 angegebene Bedeutung haben, zeigen analgetische und neuroprotektive Eigenschaften und binden mit hoher Affinität an Kappa-Rezeptoren.

## Beschreibung

Die Erfindung betrifft neue Acetamide der Formel I 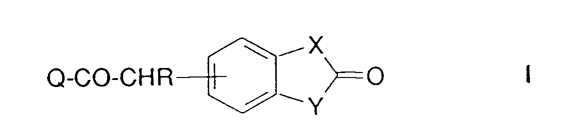
worin
Q R¹-CH(CH₂Z)-NA-, 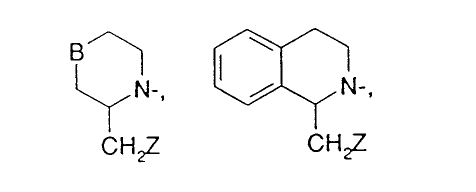 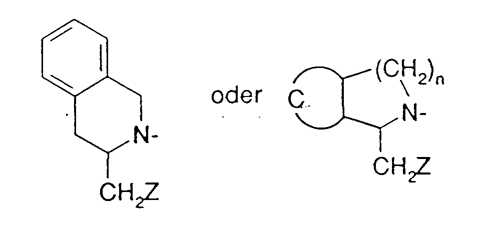
R H, A oder Ar,
X und Y jeweils unabhängig voneinander -O-, -NH-, -NA-, -CH₂-O-, -CH₂-NH- oder -CH₂-NA-,
R¹ A oder Ar,
A Alkyl mit 1 bis 6 C-Atomen,
B -O-, -NH-, -NA-, -CH₂-, -N-COA-, -N-COOA- oder eine Bindung,
C ein ankondensiertes Ringsystem mit 3 bis 5 C-Atomen, wobei gegebenenfalls ein C-Atom durch S, N oder O ersetzt sein kann und welches gegebenenfalls ein- oder zweifach durch F, Cl, Br, I, OH, OA, NH₂, NHA, NA₂, NH-COA, NA-COA oder NH-CONH₂ substituiert sein kann,
Z Pyrrolidin-1-yl oder 3-Hydroxypyrrolidin-1-yl,
Ar unsubstituiertes oder ein- oder zweifach durch A, OA oder Hal substituiertes Phenyl,
Hal F, Cl, Br oder 1 und
n 1 oder 2 bedeuten,
   sowie deren physiologisch unbedenkliche Salze.

Ähnliche Verbindungen sind aus DE-OS-42 15 213 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel 1 und ihre physiologisch unbedenklichen Salze wertvolle pharmakologische Eigenschaften besitzen. Sie zeigen eine analgetische Wirkung und antagonisieren insbesondere die entzündungsbedingte Hyperalgesie. So wirken die Verbindungen im "Writhing Test" an Mäusen oder Ratten (Methode vgl. Siegmund et al., Proc. Soc. Exp. Biol. 95, (1957), 729-731). Die analgetische Wirkung läßt sich ferner im "Tail-Flick-Test" an Mäusen oder Ratten nachweisen (Methodik vgl. d'Amour und Smith, J. Pharmacol. Exp. Ther. 72, (1941), 74-79), ferner im "Hot plate test" (vgl. Schmauss und Yaksh, J. Pharmacol. Exp. Ther. 228, (1984), 1-12 und die dort zitierte Literatur).

Besonders starke Wirkungen sind an Ratten im Modell der Carrageenininduzierten Hyperalgesie (vgl. Bartoszyk und Wild, Neuroscience Letters 101 (1989) 95) zu beobachten. Dabei zeigen die Verbindungen keine oder nur geringe Neigung zu physischer Abhängigkeit. Außerdem treten antiinflammatorische, antiasthmatische, diuretische, antikonvulsive, neuroprotektive und/oder antitussive Wirkungen auf, die ebenfalls nach hierfür geläufigen Methoden nachgewiesen werden können. Die Verbindungen zeigen eine hohe Affinität in bezug auf das Bindungsverhalten an kappaRezeptoren und wirken als L-DOPA Antagonisten. Sie eignen sich ferner zum Schutz vor und zur Behandlung von Hirnödemen und Unterversorgungszuständen des Zentralnervensystems, vor allem Hypoxie sowie zur Behandlung von Ischämien.

Die Verbindungen können daher als Arzneimittelwirkstoffe in der Human- und Veterinärmedizin verwendet werden. Ferner eignen sie sich als Zwischenprodukte zur Herstellung anderer Verbindungen mit wertvollen Eigenschaften.

Gegenstand der Erfindung sind Verbindungen der Formel 1 sowie ihre Salze.

Die Gruppe A steht für Alkyl mit 1, 2, 3, 4, 5, oder 6 C-Atomen, insbesondere für Methyl oder Ethyl, aber auch für Propyl, Isopropyl, Butyl, Isobutyl, sek.-Butyl, tert.-Butyl. Die Gruppe OA ist dementsprechend vorzugsweise Methoxy oder Ethoxy, ferner Propoxy, Isopropoxy, Butoxy, Isobutoxy, sek.-Butoxy oder tert.-Butoxy, die Gruppe -NA- vorzugsweise N-Methyl-, die Gruppe -NHA Methyl-amino und die Gruppe -NA₂ N,N-Dimethylamino.

Dementsprechend haben die nachstehenden Gruppen die im folgenden genannten bevorzugten Bedeutungen:
-NH-CO-A: Acetamido, Propionamido;
-NA-CO-A: N-Methylacetamido, N-Methylpropionamido.

Ar ist bevorzugt unsubstituiertes Phenyl, ferner bevorzugt o-, m- oder p-Methylphenyl, weiterhin bevorzugt o-, m- oder p-Methoxyphenyl, o-, m-oder p-Fluorphenyl oder o-, m- oder p-Chlorphenyl. Unter den substituierten Phenylresten sind die in p-Stellung, aber auch die in m-Stellung bevorzugt.

Der Rest R ist vorzugsweise H oder A, insbesondere Methyl.

R¹ ist besonders bevorzugt unsubstituiertes Phenyl, ferner auch p-Fluorphenyl oder p-Chlorphenyl sowie Methyl, Ethyl, Propyl oder Isopropyl.

X und Y können gleich oder verschieden voneinander sein. Sofern sie die gleiche Bedeutung haben, stehen beide Reste vorzugsweise für -NH- oder -NA-. Sind beide Gruppen verschieden voneinander, so ist einer der Reste bevorzugt -NH- oder -NA- während der andere vorzugsweise -O- oder -O-CH₂ bedeutet.

Der Rest Q besitzt vorzugsweise die folgenden Bedeutungen:
N-Methyl-N-[(1-phenyl-2-pyrrolidino)-ethyl]-amino;
N-Methyl-N-[(1-phenyl-2-(3-hydroxy-pyrrolidino))-ethyl]-amino;
N-Methyl-N-[(1-p-chlorphenyl-2-pyrrolidino)-ethyl]-amino;
N-Methyl-N-[(1-p-methoxyphenyl-2-pyrrolidino)-ethyl]-amino;
N-Methyl-N-[(1-p-methoxyphenyl-2-(3-hydroxy-pyrrolidino))-ethyl]-amino;
N-Methyl-N-(1-pyrrolidino-3-methyl-but-2-yl)-amino;
N-Methyl-N-(1 -(3-hydroxy-pyrrolidino)-3-methyl-but-2-yl)-amino;
2-(Pyrrolidino-methyl)-1,2,3,4-tetrahydroisochinolin-1-yl oder
2-(3-Hydroxy-pyrrolidino-methyl)-1,2,3,4-tetrahydroisochinolin-1-yl.

Z bedeutet Pyrrolidino, welches vorzugsweise unsubstituiert oder in 3-Position durch OH substituiert ist.

Dementsprechend sind Gegenstand der Erfindung insbesondere diejenigen Verbindungen der Formel I, in denen mindestens einer der genannten Reste eine der vorstehend angegebenen bevorzugten Bedeutungen hat.

Einige bevorzugte Gruppen von Verbindungen können durch die nachstehenden Formeln la bis le ausgedrückt werden, die der Formel 1 entsprechen und worin die nicht näher bezeichneten Reste die bei der Formel 1 angegebene Bedeutung haben, worin jedoch
in la R = H, X = -O-CH₂- und Y = -NH- bedeuten;
in Ib R = H, X = Y = -NH- oder -NA- bedeuten;
in Ic R = H, X = -O- und Y = -NH- bedeuten;
in Id R = H, X = -NH- und Y = -NA- bedeuten;
in le R = H, X = -NA- und Y = -NH- bedeuten;

Weiterhin sind bevorzugt Verbindungen der Formeln I' sowie la' bis le', die den Formeln I bzw. la bis le entsprechen, worin jedoch jeweils zusätzlich Q
(a) N-Methyl-N-[(1-phenyl-2-pyrrolidino)-ethyl]-amino;
(b) N-Methyl-N-[(1-phenyl-2-(3-hydroxy-pyrrolidino))-ethyl]-amino;
(c) N-Methyl-N-(1-pyrrolidino-3-methyl-but-2-yl)-amino;
(d) N-Methyl-N-(1-(3-hydroxy-pyrrolidino)-3-methyl)-but-2-yl)-amino;
(e) N-[(1-Phenyl-2-pyrrolidino)-ethyl]-amino;
(f) 2-(Pyrrolidino-methyl)-1,2,3,4-tetrahydroisochinolin-1-yl;
(g) 2-(3-Hydroxy-pyrrolidino-methyl)-1,2,3,4-tetrahydroisochinolin-1-yl bedeutet.

Gegenstand der Erfindung ist ferner ein Verfahren zur Herstellung von Acetamiden der Formel 1 gemäß Anspruch 1 sowie von deren Salzen, dadurch gekennzeichnet, daß man eine Verbindung der Formel II

### Q-H II

worin Q die in Anspruch 1 angegebene Bedeutung hat, mit einer Verbindung der Formel III 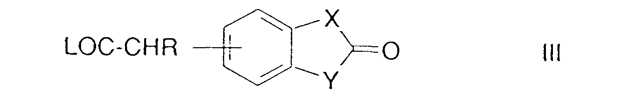
worin

L Cl, Br, OH, OA, NH₂, N₃, Acyloxy, Ar-alkoxy mit 7-11 C-Atomen oder Aroyloxy mit 6-10 C-Atomen oder eine andere reaktionsfähig veresterte OH-Gruppe bedeutet und

R, X, und Y die angegebenen Bedeutungen haben,
umsetzt,

oder daß man in einer Verbindung der Formel nach Anspruch 1 einen Rest Q, R, X und/oder Y in einen anderen Rest Q, R, X und/oder Y umwandelt,

oder daß man eine sonst der Formel 1 entsprechende Verbindung, die jedoch anstelle eines oder mehrerer Wasserstoffatome eine oder mehrere solvolysierbare Gruppe(n) enthält, mit einem solvolysierenden Mittel behandelt,

und/oder daß man eine basische Verbindung der Formel 1 durch Behandeln mit einer Säure in eines ihrer Salze umwandelt.

Die Verbindungen der Formel werden in der Regel nach an sich bekannten Methoden hergestellt, wie sie in der Literatur (z.B. in den Standardwerken wie Houben-Weyl, Methoden der Organischen Chemie, Georg-Thieme-Verlag, Stuttgart oder J. March, Adv.Org.Chem. 3rd. Ed., J. Wiley & Sons (1985)) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe sind in der Regel bekannt, oder sie können in Analogie zu bekannten Stoffen nach an sich bekannten Verfahren hergestellt werden. Sie können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die einzelnen Verfahrensvarianten werden im folgenden näher erläutert.

Die Verbindungen der Formel I sind bevorzugt herstellbar durch Reaktion der Verbindungen der Formel II mit Carbonsäuren der Formel III oder ihren funktionellen Derivaten. Als funktionelle Derivate der Verbindungen der Formel III eignen sich insbesondere die entsprechenden Ester, vor allem die Methyl-oder Ethylester, die Halogenide, Anhydride oder Azide; die Chloride sind bevorzugt.

Verbindungen der Formel II sind beispielsweise erhältlich durch Umsetzung von 1-(Chlormethyl)-1,2,3,4-tetrahydroisochinolin mit Pyrrolidin oder 3-Hydroxypyrrolidin, von 1-Amino-1-phenyl-2-pyrrolidino-ethan mit Methyliodid, von 1-N-Methylamino-1-phenyl-2-halogen-ethan (Halogen entspricht vorzugsweise CI od. Br) mit Pyrrolidin oder 3-Hydroxypyrrolidin oder von 1-Halogen-2-N-methylamino-4-methyl-pentan mit Pyrrolidin oder dessen 3-Hydroxyderivat.

Ferner kann man Verbindungen der Formel II auch durch Umsetzung von 2-Halogenmethylderivaten des Piperazins oder Piperidins mit Pyrrolidin oder 3-Hydroxypyrrolidin erhalten.

Typische Verbindungen der Formel III sind z.B. Essigsäure- oder Phenylessigsäure-Derivate, wie beispielsweise Chloride, Bromide, Azide, Methyl- oder Ethylester oder Anhydride, die zusätzlich in 2-Stellung mit einem Rest der Formel Illa 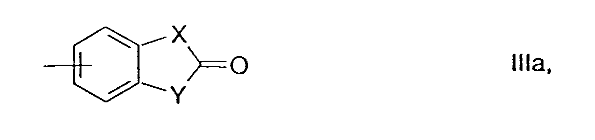 wobei X und Y die angegebenen Bedeutungen haben, verbunden sind.

Herstellbar sind die Verbindungen der Formel III beispielsweise durch Umsetzung von 3-, 4-, 5- oder 6-Halogenmethyl-2-amino-phenolen oder den entsprechenden 3-, 4-, 5- oder 6-Halogenmethyl-o-phenylendiamin-Derivatenmit Phosgen oder anderen reaktiven Kohlensäurederivaten unter an sich bekannten Bedingungen und anschließende Überführung des Halogenrestes in eine Carboxylgruppe bzw. ein daraus herstellbares Derivat, wie z.B. ein Säurechlorid. Weiterhin gelingt die Herstellung von Verbindungen der Formel 1 beispielsweise durch Umsetzung von 3-Amino-4-hydroxyphenylessigsäure oder 3,4-Diaminophenylessigsäure mit 1,1'-Carbonyldümidazol.

Die Umsetzung von 11 mit III bzw. III-Derivaten gelingt zweckmäßig in Anwesenheit oder Abwesenheit eines inerten organischen Lösungsmittels, z.B. eines halogenierten Kohlenwasserstoffs wie Dichlormethan, Chloroform oder Trichlorethen, eines Alkohols wie Methanol, Ethanol oder Butanol, eines Ethers wie Tetrahydrofuran (THF) oder Dioxan, eines Amids wie Dimethylformamid (DMF), eines Sulfoxids wie Dimethylsulfoxid (DMSO) und/oder in Gegenwart oder Abwesenheit eines Kondensationsmittels, z.B. einer Base, bei Temperaturen zwischen -20 und 200 °C, vorzugsweise 0 und 100 °C. Als Basen eignen sich z.B. Alkalimetallhydroxide wie NaOH oder KOH, Alkalimetallcarbonate wie Na₂C0₃ oder K₂C0₃, tertiäre Amine wie Triethylamin oder Pyridin. Als Lösungsmittel ist Dichlormethan, als Base Triethylamin besonders bevorzugt.

Ferner kann man in einer Verbindung der Formel 1 einen oder mehrere der Reste Q, R, X und/oder Y in einen oder mehrere andere Reste Q, R, X und/oder Y umwandeln.

So kann man Ethergruppen (z.B. OA-Gruppen) oder Estergruppen unter Bildung von OH-Gruppen Spalten, z.B. durch Behandeln mit Dimethylsulfid-Bortribromid-Komplex, z.B. in Toluol, THF oder DMSO, oder durch Verschmelzen mit Pyridin- oder Anilinhydrohalogeniden, vorzugsweise Pyridinhydrochlorid, bei etwa 150-250 °C, oder durch Behandeln mit Diisobutylaluminiumhydrid in Toluol bei etwa 0-110 _{°} C.

Weiterhin kann man OH-Gruppen verethern oder verestern, z.B. indem man zunächst die entsprechenden Alkalimetall- (z.B. Na- oder K-)alkoholate, -phenolate oder andere Salze herstellt und diese mit entsprechenden Halogenverbindungen umsetzt, z.B. mit Alkylhalogeniden wie Methylchlorid, -bromid oder -iodid, Chlor- oder Bromacetamid, zweckmäßig in Gegenwart eines der oben angegebenen Lösungsmittel bei Temperaturen zwischen 0 und 100 °C.

Nitrogruppen können zu Aminogruppen reduziert werden, zweckmäßig durch katalytische Hydrierung unter den oben angegebenen Bedingungen, z.B. mit Raney-Ni im Methanol oder Ethanol bei 15-40 und Normaldruck.

Aminogruppen können acyliert werden, z.B. mit Säurechloriden wie Acetyl-, Methansulfonyl-, Oxalsäure- oder Bernsteinsäurehalbesterchlorid, zweckmäßig in inerten Lösungsmitteln wie Dichlormethan bei 15-40_{°}.

Ferner können Aminogruppen nach an sich bekannten Methoden alkyliert werden.

Außerdem können Verbindungen, die sonst der Formel I entsprechen, aber anstelle eines oder mehrerer H-Atome eine oder mehrere solvolysierbare Gruppe(n) enthalten, zu Verbindungen der Formel I solvolysiert, insbesondere hydrolysiert werden.

So können insbesondere 3-Acylbenzoxazol-Derivate (entsprechend der Formel I, aber in 3-Stellung des Benzoxazols eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 3-Stellung des Benzoxazol-Ringes unsubstituierten Benzoxazols hydrolysiert werden, z.B. in saurem, besser in neutralem oder alkalischem Medium bei Temperaturen zwischen 0 und 200 _{°}C. Als Basen verwendet man zweckmäßig Natrium-, Kalium- oder Calciumhydroxid, Natrium- oder Kaliumcarbonat oder Ammoniak Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Eine Base der Formel kann weiterhin mit einer Säure in das zugehörige Säureadditionssalz überführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Aufreinigung der Verbindungen der Formel verwendet werden.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel 1 und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Trägeroder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen, insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks-und/oder Aromastoffe enthalten. Sie können, falls erwünscht, auch einen oder mehrere weitere Wirkstoffe enthalten, z.B. ein oder mehrere Vitamine.

Die Verbindungen der Formel 1 und ihre physiologisch unbedenklichen Salze können bei der Bekämpfung von Krankheiten, insbesondere von Schmerzzuständen aber auch zur Minderung der Folgeschäden nach einer Ischämie, vorzugsweise einer cerebralen Ischämie verwendet werden.

Dabei werden die erfindungsgemäßen Substanzen in der Regel in Analogie zu bekannten Analgetika wie z.B. Tramadol verabreicht, vorzugsweise in Dosierungen zwischen etwa 1 und 500 mg, insbesondere zwischen 5 und 100 mg pro Dosierungseinheit. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und - weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in _{°} C angegeben. Einige Verbindungen der Formel I neigen bei Erhitzen zur Zersetzung, so daß keine eindeutigen Schmelzpunkte ermittelt werden konnten. Deshalb werden in diesen Fällen, sofern es möglich ist, ersatzweise die entsprechenden Rf-Werte (Dünnschichtchromatographie) angegeben. In den nachfolgenden Beispielen bedeutet "übliche Aufarbeitung": Man gibt, falls erforderlich, Wasser oder verdünnte Natronlauge hinzu, extrahiert mit Dichlormethan, trennt ab, trocknet die organische Phase mit Natriumsulfat, filtriert, dampft ein und reinigt durch Chromatographie an Kieselgel und/oder durch Kristallisation.

### Beispiel 1

Eine Suspension von 3,9 g 2-(2,3-Dihydro-2-oxobenzoxazol-5-yl)-essigsäure [erhältlich durch Umsetzung von 3-Amino-4-hydroxy-phenyl-essigsäuremethylester mit 1,1'-Carbonyldümidazol und anschließende Verseifung] in 40 ml Toluol wird mit 1,6 ml Thionylchlorid versetzt und unter Rühren 1 Std. gekocht. Anschließend wird 1 ml DMF zugegeben und weitere 15 Min. erhitzt. Nach Entfernung des Lösungsmittels wird der Rückstand in 25 ml THF aufgenommen und tropfenweise zu einer Lösung von 4 g Triethylamin und 4,4 g (1S)-[1-N-Methyl-amino-1-phenyl-2-((3S)-3-hydroxy-pyrrolidino)]-ethan [erhältlich aus (1S)-1-Amino-1-phenyl-2-chlorethan durch Umsetzung mit (3S)-3-Hydroxypyrrolidin und anschließende Methylierung mit Methyliodid] in 40 ml THF gegeben. Nach zweistündigem Rühren wird das Lösungsmittel entfernt und wie üblich aufgearbeitet. Man erhält N-Methyl-N-[(1S)-1-phenyl-2-((3S)-3-hydroxy-pyrrolidino)-ethyl]-2-(2,3-dihydro-2-oxobenzoxazol-5-yl)-acetamid, F. 207-2080.

Analog erhält man durch Umsetzung von 2-(2,3-Dihydro-2-oxobenzoxazol-5-yl)-essigsäure mit (1S)-(1-N-Methyl-amino-1-phenyl-2-pyrrolidino)-ethan:
N-Methyl-N-[(1 S)-1-phenyl-2-pyrrolidino-ethyl]-2-(2,3-dihydro-2-oxobenzoxazol-5-yl)-acetamid; mit (1S)-[1-N-Methyl-amino-1-p-methoxyphenyl-2-((3S)-3-hydroxypyrrolidino)]-ethan:
N-Methyl-N-[(1 S)-1-p-methoxyphenyl-2-((3S)-3-hydroxy-pyrrolidinoethyl]-2-(2,3-dihydro-2-oxobenzoxazol-5-yl) -acetamid;
mit 1-(Pyrrolidino-methyl)-1,2,3,4-tetrahydroisochinolin:
1-(Pyrrolidino-methyl)-2-[(2,3-dihydro-2-oxobenzoxazol-5-yl)-acetyl]-1,2,3,4-tetrahydroisochinolin; mit 1-[(3S)-3-Hydroxy-pyrrolidino-methyl]-1,2,3,4-tetrahydroisochinolin:
1-[(3S)-3-Hydroxypyrrolidino-methyl]-2-[(2,3-dihydro-2-oxo-benzoxazol-5-yl)-acetyl]-1,2,3,4-tetrahydroisochinolin, F. 130-135° (Zers.);
mit 3-[(3S)-3-Hydroxy-pyrrolidino-methyl]-1,2,3,4-tetrahydroisochinolin:
2-[(2,3-Dihydro-2-oxo-benzoxazol-5-yl)-acetyl]-3-[(3S)-3-hydroxypyrrolidino-methyl]--1,2,3,4-tetrahydroisochinolin, F. 179°;
mit 1-(Pyrrolidino-methyl)-1,2,3,4-tetrahydroisochinolin:
1-(Pyrrolidino-methyl)-2-[(2,3-dihydro-2-oxo-benzoxazol-5-yl)-acetyl]-1,2,3,4-tetrahydroisochinolin; mit 3-(Pyrrolidino-methyl)-1,2,3,4-tetrahydroisochinolin:
2-[(2,3- Dihydro-2-oxo-benzoxazol-5-yl)-acetyl]-3-(pyrrolidino-methyl)-1,2,3,4-tetrahydroisochinolin; mit N-Methyl-N-[(2S)-1-pyrrolidino-3-methyl-but-2-yl]-amin:
N-Methyl-N-[(2S)-1-pyrrolidino-3-methyl-but-2-yl]-2-(2,3-dihydro-2-oxobenzoxazol-5-yl)-acetamid; mit N-Methyl-N-[2(S)-1-((3S)-3-hydroxy-pyrrolidino)-3-methyl-but-2-yl]-amin:
N-Methyl-N-[(2S)-1-((3S)-3-hydroxy-pyrrolidino)-3-methyl-but-2-yl]-2-(2,3-dihydro-2-oxobenzoxazol-5-yl)-acetamid (Öl), Rf: 0,45 (Kieselgel/CH₂/CH₃0H 9:1; 1 % NH₃);

### Beispiel 2

Analog Beispiel 1 erhält man durch Umsetzung von 2-(3-Oxo-3,4-dihydro-2H-1,4-benzoxazin-6-yl)-essigsäure
mit (1S)-[1-N-Methyl-amino-1-phenyl-2-((3S)-3-hydroxy-pyrrolidino)]-ethan: N-Methyl-N-[(1 S)-1-phenyl-2-((3S)-3-hydroxy-pyrrolidino)-ethyl]-2-(3-oxo-3,4-dihydro-2H-1,4-benzoxazin-6-yl)-acetamid-hydrochlorid, F. 237-239°;
mit (1S)-(1-N-Methyl-amino-1-phenyl-2-pyrrolidino)-ethan:
N-Methyl-N-[(1 S)-1-phenyl-2-pyrrolidino-ethyl]-2-(3-oxo-3,4-dihydro-2H-1,4-benzoxazin-6-yl)-acetamid; mit (1S)-[1-N-Methyl-amino-1-p-methoxyphenyl-2-((3S)-3-hydroxy-pyrrolidino)]-ethan:
N-Methyl-N-[(1 S)-1-p-methoxyphenyl-2-((3S)-3-hydroxy-pyrrolidinoethyl]-2-(3-oxo-3,4-dihydro-2H-1,4-benzoxazin-6-yl)-acetamid;
mit 1-(Pyrrolidino-methyl)-1,2,3,4-tetrahydroisochinolin:
1-(Pyrrolidino-methyl)-2-[(3-oxo-3,4-dihydro-2H-1,4-benzoxazin-6-yl)-acetyl]-1,2,3,4-tetrahydroisochinolin; mit 1-[(3S)-3-Hydroxy-pyrrolidino-methyl]-1,2,3,4-tetrahydro-isochinolin:
1-[(3S)-3-Hydroxypyrrolidino-methyl]-2-[(3-oxo-3,4-dihydro-2H-1,4-benzoxazin-6-yl)-acetyl]1,2,3,4-tetrahydroisochinolin;
mit 3-[(3S)-3-Hydroxy-pyrrolidino-methyl]-1,2,3,4-tetrahydro-isochinolin:
2-[(3-Oxo-3,4-dihydro-2H-1,4-benzoxazin-6-yl)-acetyl]-3-[(3S)-3-hydroxypyrrolidino-methyl]--1,2,3,4-tetrahydroisochinolin;
mit 1-(Pyrrolidino-methyl)-1,2,3,4-tetrahydroisochinolin:
1-(Pyrrolidino-methyl)-2-[(3-oxo-3,4-dihydro-2H-1,4-benzoxazin-6-yl)-acetyl]-1,2,3,4-tetrahydroisochinolin; mit 3-(Pyrrolidino-methyl)-1,2,3,4-tetrahydroisochinolin:
2-[(3-Oxo-3,4-dihydro-2H-1,4-benzoxazin-6-yl)acetyl]-3-(pyrrolidinomethyl)--1,2,3,4-tetrahydroisochinolin; mit N-Methyl-N-[(2S)-1-pyrrolidino-3-methyl-but-2-yl]-amin:
N-Methyl-N-[(2S)-1-pyrrolidino-3-methyl-but-2-yl]-2-(3-oxo-3,4-dihydro-2H-1,4-benzoxazin-6-yl)-acetamid;
mit N-Methyl-N-[2(S)-1-((3S)-3-hydroxy-pyrrolidino)-3-methyl-but-2-yl]-amin:
N-Methyl-N-[(2S)-1-((3S)-3-hydroxy-pyrrolidino)-3-methyl-but-2-yl]-2-(3-oxo-3,4-dihydro-2H-1,4-benzoxazin-6-yl)-acetamid.

### Beispiel 3

Analog Beispiel 1 erhält man durch Umsetzung von 2-(2-Oxo-2,3-dihydrobenzimidazol-5-yl)-essigsäure mit (1S)-[1-N-Methyl-amino-1-phenyl-2-((3S)-3-hydroxy-pyrrolidino)]-ethan:
N-Methyl-N-[(1 S)-1-phenyl-2-((3S)-3-hydroxy-pyrrolidino)-ethyl]-2-(2-oxo-2,3-dihydro-benzimidazol-5-yl)-acetamid -hydrochlorid, F. 161 °;
mit (1S)-(1-N-Methyl-amino-1-phenyl-2-pyrrolidino)-ethan: N-Methyl-N-[(1S)-1-phenyl-2-pyrrolidino-ethyl]-2-(2-oxo-2,3-dihydrobenzimidazol-5-yl)-acetamid, F. 190°;
mit (1S)-[1-N-Methyl-amino-1-p-methoxyphenyl-2-((3S)-3-hydroxypyrrolidino)]-ethan:
N-Methyl-N-[(1 S)-1-p-methoxyphenyl-2-((3S)-3-hydroxy-pyrrolidinoethyl]-2-(2-oxo-2,3-dihydro- benzimidazol-5-yl)-acetamid;
mit 1-(Pyrrolidino-methyl)-1,2,3,4-tetrahydroisochinolin:
1-(Pyrrolidino-methyl)-2-[(2-oxo-2,3-dihydro-benzimidazo-5-yl)-acetyl]-1,2,3,4-tetrahydroisochinolin; mit 1-[(3S)-3-Hydroxy-pyrrolidino-methyl]-1,2,3,4-tetrahydro-isochinolin:
1-[(3S)-3-Hydroxypyrrolidino-methyl]-2-[(2-oxo-2,3-dihydrobenzimidazol-5-yl)-acetyl]-1,2,3,4-tetrahydroisochinolin ;
mit 3-[(3S)-3-Hydroxy-pyrrolidino-methyl]-1,2,3,4-tetrahydro-isochinolin:
2-[(2-Oxo-2,3-dihydro-benzimidazol-5-yl)-acetyl]-3-[(3S)-3-hydroxypyrrolidino-methyl]--1,2,3,4-tetrahydroisochinolin;
mit 1-(Pyrrolidino-methyl)-1,2,3,4-tetrahydroisochinolin:
1-(Pyrrolidino-methyl)-2-[(2-oxo-2,3-dihydro-benzimidazol-5-yl)-acetyl]-1,2,3,4-tetrahydroisochinolin; mit 3-(Pyrrolidino-methyl)-1,2,3,4-tetrahydroisochinolin:
2-[(2-Oxo-2,3-dihydro-benzimidazol-5-yl)-acetyl]-3-(pyrrolidinomethyl)--1,2,3,4-tetrahydroisochinolin; mit N-Methyl-N-[(2S)-1-pyrrolidino-3-methyl-but-2-yl]-amin:
N-Methyl-N-[(2S)-1-pyrrolidino-3-methyl-but-2-yl]-2-(2-oxo-2,3-dihydro-benzimidazol-5-yl)-acetamid; mit N-Methyl-N-[2(S)-1-((3S)-3-hydroxy-pyrrolidino)-3-methyl-but-2-yl]-amin:
N-Methyl-N-[(2S)-1-((3S)-3-hydroxy-pyrrolidino)-3-methyl-but-2-yl]-2-(2-oxo-2,3-dihydro-benzimidazol-5-yl)-acetamid.

### Beispiel 4

Analog Beispiel 1 erhält man durch Umsetzung von 2-(1-Methyl-2-oxo-2,3-dihydro-benzimidazol-5-yl)-essigsäure mit (1S)-[1-N-Methyl-amino-1-phenyl-2-((3S)-3-hydroxy-pyrrolidino)]-ethan:
N-Methyl-N-[(1 S)-1-phenyl-2-((3S)-3-hydroxy-pyrrolidino)-ethyl]-2-(1-methyl-2-oxo-2,3-dihydro- benzimidazol-5-yl) -acetamid, F. 220°;
mit (1S)-(1-N-Methyl-amino-1-phenyl-2-pyrrolidino)-ethan:
N-Methyl-N-[(1 S)-1-phenyl-2-pyrrolidino-ethyl]-2-(1-methyl-2-oxo-2,3-dihydro-benzimidazol-5-yl)-acetamid, F. 278°;
mit (1S)-[1-N-Methyl-amino-1-p-methoxyphenyl-2-((3S)-3-hydroxypyrrolidino)]-ethan:
N-Methyl-N-[(1 S)-1-p-methoxyphenyl-2-((3S)-3-hydroxy-pyrrolidinoethyl]-2-(1-methyl-2-oxo-2,3-dihydro- benzimidazol-5-yl)-acetamid;
mit 1-(Pyrrolidino-methyl)-1,2,3,4-tetrahydroisochinolin:
1 -(Pyrrolidino-methyl)-2-[(1-methyl-2-oxo-2,3-dihydro-benzimidazol-5-yl)-acetyl]-1,2,3,4-tetrahydroisochinolin;
mit 1-[(3S)-3-Hydroxy-pyrrolidino-methyl]-1,2,3,4-tetrahydro-isochinolin:
1-[(3S)-3-Hydroxypyrrolidino-methyl]-2-[(1-methyl-2-oxo-2,3-dihydrobenzimidazol-5-yl)-acetyl]-1,2,3,4-tetrahydro-isochinolin;
mit 3-[(3S)-3-Hydroxy-pyrrolidino-methyl]-1,2,3,4-tetrahydro-isochinolin:
2-[(1-Methyl-2-oxo-2,3-dihydro-benzimidazol-5-yl)-acetyl]-3-[(3S)-3-hydroxypyrrolidino-methyl]--1,2,3,4-tetrahydro-isochinolin;
mit 1-(Pyrrolidino-methyl)-1,2,3,4-tetrahydroisochinolin:
1 -(Pyrrolidino-methyl)-2-[(1-methyl-2-oxo-2,3-dlhydro-benzimidazol-5-yl)-acetyl]-1,2,3,4-tetrahydroisochinolin;
mit 3-(Pyrrolidino-methyl)-1,2,3,4-tetrahydroisochinolin:
2-[(1-methyl-2-oxo-2,3-dihydro-benzimidazol-5-yl)-acetyl]-3-(pyrrolidino-methyl)--1,2,3,4-tetrahydroisochinolin;
mit N-Methyl-N-[(2S)-1-pyrrolidino-3-methyl-but-2-yl]-amin:
N-Methyl-N-[(2S)-1-pyrrolidino-3-methyl-but-2-yl]-2-(1-methyl-2-oxo-2,3-dihydro-benzimidazol-5-yl)-acetamid;
mit N-Methyl-N-[2(S)-1-((3S)-3-hydroxy-pyrrolidino)-3-methyl-but-2-yl]-amin:
N-Methyl-N-[(2S)-1-((3S)-3-hydroxy-pyrrolidino)-3-methyl-but-2-yl]-2-(1-methyl-2-oxo-2,3-dihydro-benzimidazol-5-yl)-acetamid.

### Beispiel 5

Man löst 3,1 g 2-(3-Oxo-3,4-dihydro-2H-1,4-benzoxazin-6-yl)-essigsäurehydrazid [z.B. erhältlich aus dem entsprechenden Ethylester durch Umsetzung mit Hydrazin] in 200 ml stark verdünnter Salzsäure, tropft unter Rühren bei 0° eine Lösung von 2,0g NaN0₂ in 40 ml Wasser hinzu, rührt 30 Min. und extrahiert das gebildete Azid mit Dichlormethan. Nach Trocknen über MgS0₄ und Konzentrieren auf 50 ml wird das so erhaltene Reagenz zu einer Lösung von (1S)-[1-N-Methyl-amino-1-(2,4-dichlorphenyl)-2-((3S)-3-hydroxy-pyrrolidino)]-ethan und 4 ml Triethylamin in 100 ml Dichlormethan zugetropft. Man rührt noch 2 Std. bei Raumtemperatur und erhält nach üblicher Aufarbeitung N-Methyl-N-[(1S)-1-(2,4-dichlorphenyl)-2-(-(3S)-3-hydroxy-pyrrolidino)-ethyl]-2-(3-oxo-3,4-dihydro-2H-1,4-benzoxazin-6-yl)-acetamid.

### Beispiel 6

Man hydriert eine Lösung von 1 g N-Methyl-N-[(1S)-1-p-benzyloxy-phenyl-2-((3S)-3-benzyloxy-pyrrolidino)-ethyl]-2-(2-oxo-2,3-dihydro-benzimidazol-5-yl)-acetamid in 25 ml Ethylacetat an 0,5 g 5%iger Pd-C bei 20° und 1 bar bis zum Stillstand der Wasserstoffaufnahme, filtriert, dampft ein und erhält N-Methyl-N-[(1 S)-1-p-hydroxy-phenyl-2-((3S)-3-hydroxy-pyrrolidino)-ethyl]-2-(2-oxo-2,3-dihydro-benzimidazol-5-yl)-acetamid.

### Beispiel 7

Man löst 3,2 g N-[(1S)-1-Phenyl-2-((3S)-3-hydroxy-pyrrolidino)-ethyl]-2-(2-oxo-2,3-dihydrobenzimidazol-5-yl)-acetamid in 150 ml Dichlormethan und tropft unter Rühren 3 Äquivalente Methyliodid, gelöst in 20 ml Dichlormethan, hinzu, konzentriert die Lösung und erhält nach üblicher Aufarbeitung N-Methyl-N-[(1S)-1-phenyl-2-((3S)-3-hydroxy-pyrrolidino)-ethyl]-2-(1,3-dimethyl-2-oxo-2,3-dihydrobenzimidazol-5-yl)-acetamid.

### Beispiel 8

0,9 g N-Methyl-N-[(1S)-1-phenyl-2-((3S)-3-hydroxy-pyrrolidino)-ethyl]-2-(2,3-dihydro-2-oxobenzoxazol-5-yl)-acetamid werden in 50 ml ethanolischer HCI-Lösung gelöst und 6 Std. bei Raumtemperatur gerührt. Nach Entfernung des Lösungsmittels und Waschen mit wenig Ethanol erhält man nach Trocknung N-Methyl-N-[(1 S)-1-phenyl-2-((3S)-3-hydroxy-pyrrolidino)-ethyl]-2-(2,3-dihydro-2-oxobenzoxa-zol-5-yl)-acetamid-hydrochlorid, F. 263°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄•2H₂O, 28,48 g Na₂HPO₄•12H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Acetamide der Formel I 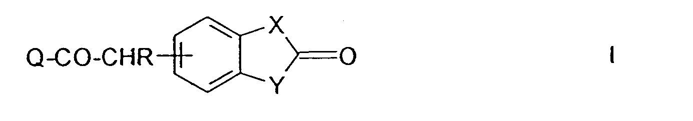
worin
Q R¹-CH(CH₂Z)-NA-, 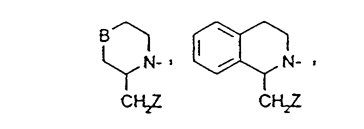 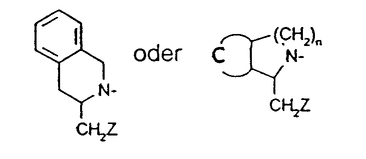
R H, A oder Ar,
X und Y jeweils unabhängig voneinander -O-,-NH-, -NA-, -CH₂-O-, -CH₂-NH- oder -CH₂-NA-, R¹ A oder Ar
A Alkyl mit 1 bis 6 C-Atomen,
B -O-, -NH-, -NA-, -CH₂-, -N-COA-, -N-COOA- oder eine Bindung,
C ein ankondensiertes Ringsystem mit 3 bis 5 C-Atomen, wobei gegebenenfalls ein C-Atom durch S, N oder O ersetzt sein kann und welches gegebenenfalls ein- oder zweifach durch F, Cl, Br, I, OH, OA, NH₂, NHA, NA₂, NH-COA, NA-COA oder NH-CONH₂ substituiert sein kann,
Z Pyrrolidin-1-yl oder 3-Hydroxypyrrolidin-1-yl,
Ar unsubstituiertes oder ein- oder zweifach durch A, OA oder Hal substituiertes Phenyl, Hal F, Cl, Br oder 1 und
n 1 oder 2 bedeuten,
sowie deren physiologisch unbedenkliche Salze.

2.
(a) N-Methyl-N-[(1S)-1-phenyl-2-((3S)-3-hydroxy-pyrrolidino)-ethyl]-2-(2,3-dihydro-2-oxobenzoxazol-5-yl)-acetamid;
(b) N-Methyl-N-(2S)-[1-((3S)-3-hydroxy-pyrrolidino-3-methyl-but-2-yl]-2-(2,3-dihydro-2-oxobenzoxa- zol-5-yl)-acetamid;
(c) 2-(2,3-Dihydro-2-oxobenzoxazol-5-yl)-acetyl-1-[(3S)-3-hydroxypyrrolidino-methyl)-1,2,3,4-tetrahydroisochinolin;
(d) 2-(2,3-Dihydro-2-oxobenzoxazol-5-yl)-acetyl-3-[(3S)-3-hydroxypyrrolidino-methyl)-1,2,3,4-tetrahydroisochinolin;
(e) N-Methyl-N-[(1S)-1-phenyl-2-((3S)-3-hydroxy-pyrrolidino)-ethyl]-2-(2,3-dihydro-2-oxobenzimidazol-5-yl)-acetamid;
(f) N-Methyl-N-[(1S)-1-phenyl-2-((3S)-3-hydroxy-pyrrolidino)-ethyl]-2-(2,3-dihydro-1-methyl-2-oxoben- zimidazol-5-yl)-acetamid;
(g) N-Methyl-N-[(1 S)-1 -phenyl-2-pyrrolidino-ethyl]-2-(2,3-dihydro-1-methyl-2-oxobenzimidazol-5-yl)-acetamid.

3. Verfahren zur Herstellung von Acetamiden der Formel 1 sowie von deren Salzen, dadurch gekennzeichnet, daß man eine Verbindung der Formel II
Q-H II,
worin Q die in Anspruch 1 angegebene Bedeutung hat, mit einer Verbindung der Formel III 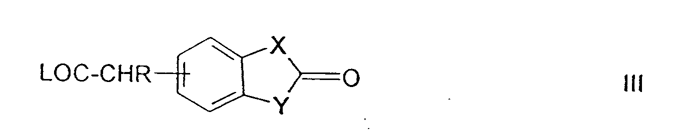
worin
L Cl, Br, OH, OA, NH₂, N₃, Acyloxy, Ar-alkoxy mit 7-11 C-Atomen oder Aroyloxy mit 6-10 C-Atomen oder eine andere reaktionsfähig veresterte OH-Gruppe bedeutet und
R, X und Y die angegebenen Bedeutungen haben, umsetzt,
oder daß man in einer Verbindung der Formel I nach Anspruch 1 einen Rest Q, R, X und/oder Y in einen anderen Rest Q, R, X und/oder Y umwandelt,
oder daß man eine sonst der Formel 1 entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man eine basische Verbindung der Formel I durch Behandeln mit einer Säure in eines ihrer Salze umwandelt.

4. Verfahren zur Herstellung einer pharmazeutischen Zubereitung, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Darreichungsform bringt.

5. Pharmazeutische Zubereitung, gekennzeichnet durch einen Gehalt an mindestens einer Verbindung der Formel I, gemäß Anspruch 1, und/oder einem ihrer physiologisch unbedenklichen Salze.

6. Verwendung einer Verbindung der Formel I gemäß Anspruch 1 und/oder eines ihrer physiologisch unbedenklichen Salze bei der Bekämpfung von Krankheiten.

7. Verwendung einer Verbindung der Formel I gemäß Anspruch 1 und/oder eines ihrer physiologisch unbedenklichen Salze zur Herstellung von Arzneimitteln.
